# EUROPEAN PATENT APPLICATION

(11) **EP 4 197 443 A1**
(43) Date of publication of application: **21.06.2023**
(21) Application number: 21215009.8
(22) Date of filing: 16.12.2021
(51) Int. Cl.: A61B 6/00

(54) **X-RAY IMAGE PROCESSING SYSTEM, X-RAY IMAGING SYSTEM AND METHOD FOR PROCESSING AN X-RAY IMAGE**

(71) Applicant: Koninklijke Philips N.V., 5656 AG Eindhoven (NL)
(72) Inventor: BYSTROV, Daniel, Eindhoven (NL); FRERKING, Lena, Eindhoven (NL); KROENKE, Sven, Eindhoven (NL); GOOßEN, André, Eindhoven (NL); MAY, Jan Marek, Eindhoven (NL); VON BERG, Jens, Eindhoven (NL); YOUNG, Stewart, Eindhoven (NL); BRUECK, Heiner Matthias, Eindhoven (NL)
(74) Representative: Philips Intellectual Property & Standards

(57) **Abstract**

The invention relates to an X-ray image processing system (4) comprising an X-ray image input interface (8), a computing unit (9) and an X-ray image output interface (11). The X-ray image input interface (8) is configured to receive original X-ray images taken by an X-ray imaging system (1) comprising an X-ray source (2) and an X-ray detector (3). The computing unit (9) is connected to the X-ray image input interface (8) and is configured to obtain at least one original X-ray image from the X-ray image input interface (8), obtain information relating to the relative detector pose of the X-ray detector (3) relative to the X-ray source (2) and obtain a point of interest (P) in the original X-ray image. The computing unit (9) is further configured to apply a perspective transformation to the original X-ray image to obtain a transformed X-ray image, wherein the transformed X-ray image corresponds to a perspective where the X-ray detector (3) is perpendicular to an X-ray beam (6) from the X-ray source (2) to the point of interest (P). Finally, the X-ray image output interface (11) is configured to transmit the transformed X-ray image.

## Description

### FIELD OF THE INVENTION

The invention relates to an X-ray image processing system, to an X-ray imaging system with an X-ray image processing system and to a method for processing an X-ray image.

### BACKGROUND OF THE INVENTION

Detector positioning is one of the challenging tasks in X-ray image acquisition, especially for free projections used for musculo-skeletal exams or in bed-side chest exams frequently taken for monitoring patient health in intensive care units. The radiographer wants to position the X-ray detector beside or underneath the anatomical structure of the patient to be imaged so that the central beam of the tube passes the centre of this structure, e.g., a joint centre, and then hits the detector plane perpendicularly.

Apart from the orthogonal beam from the tube to the detector, X-ray images always comprise perspective distortions. The location of the minimal perspective distortion is the position, where the orthogonal beam hits the detector. It is desirable that this position is close to the centre of the imaged anatomical structure, e.g., the joint centre. Nevertheless, for various reasons, this cannot always be achieved such that after an acquisition the resulting X-ray images reveal perspective distortions close to the anatomical structure of interest. This is caused by a tilt of the detector relative to the central beam. In extreme cases, this will be a reason to retake an X-ray image, which causes additional radiation for the patient.

### SUMMARY OF THE INVENTION

It is an object of the present invention to provide an X-ray image processing system that corrects the perspective distortions. It is a further object of the present invention to provide an X-ray imaging system with an X-ray image processing system and a method for processing an X-ray image that correct the perspective distortions.

In an aspect of the present invention, an X-ray image processing system is provided. The X-ray image processing system comprises an X-ray image input interface, a computing unit and an X-ray image output interface. The X-ray image input interface and the X-ray image output interface may be integrated in the computing unit and/or they may be wired or wirelessly connected to the computing unit.

The X-ray image input interface is configured to receive original X-ray images taken by an X-ray imaging system. Said X-ray imaging system comprises an X-ray source and an X-ray detector. In operation, X-ray beams emitted by the X-ray source pass an object of interest, in particular an anatomical structure of a patient, and are recorded by the X-ray detector to generate the original X-ray image.

The computing unit is connected to the X-ray image input interface and obtains at least one original X-ray image from the X-ray image input interface. Here, the X-ray image input interface may receive the original X-ray image directly from the X-ray detector, may receive the original X-ray image from an image preprocessor or may read an X-ray image file already saved to a memory.

Further, the computing unit obtains information relating to the relative detector pose of the X-ray detector relative to the X-ray source. In this context, "pose" is a combination of position and orientation.

Also, the computing unit obtains a point of interest in the original X-ray image. Said point of interest may be indicated by its coordinates within the original X-ray image. The point of interest may be a certain anatomical structure or a certain part within an anatomical structure, such as a center of a joint.

Using the information relating to the relative detector pose and the point of interest, the computing unit applies a perspective transformation to the original X-ray image to obtain a transformed X-ray image such that the transformed X-ray image corresponds to a perspective where the X-ray detector is perpendicular to an X-ray beam from the X-ray source to the point of interest. In said transformed X-ray image, perspective distortions are minimized at the point of interest and in a region surrounding the point of interest.

Hence, even if the original X-ray image features perspective distortions at the point of interest, said perspective distortions are corrected by the X-ray image processing system. In particular, there is no need to retake an X-ray image because of perspective distortions at the point of interest.

Finally, the X-ray image output interface obtains and transmits the transformed X-ray image.

According to an embodiment, the information relating to the relative detector pose comprises a relative position and a relative orientation of the X-ray detector relative to the X-ray source. Said relative position and relative orientation may be provided in a coordinate system fixed to the X-ray source. The relative position and the relative orientation are the full information for performing a perspective transformation. However, good perspective transformations may already be achieved knowing the relative orientation and the approximate distance between the X-ray source and the X-ray detector. Also, there exist other equivalent descriptions of the information relating to the relative detector pose, such as the coordinates at least three of the corners of the X-ray detector in the coordinate frame fixed to the X-ray source. Of course, also other corrdinate frames may be used. In that case, a position of the X-ray source is usually also required.

According to an embodiment, the information relating to the relative detector pose has been provided by external input. This is particularly relevant for X-ray imaging systems with a fixed position of the X-ray source and the X-ray detector, such that a correct determination of the relative detector pose can be performed once and then be provided to the X-ray image processing system.

According to an embodiment, the computing unit is configured to interactively obtain the information relating to the relative detector pose, in particular a direction and an amount of an X-ray detector tilt, by user input. Said user input may be provided, for example, by a pointing device, by touching a touch screen or by a gesture. Here, the user, in particular a radiologist, uses his experience to determine for which relative detector pose the perspective distortion is minimized at the point of interest. The input itself does not necessarily have to be exactly the relative detector pose but may be an intuitive input that relates to tilt direction and tilt amount.

According to an embodiment, the information relating to the relative detector pose is obtained by performing an analysis of a calibration X-ray image taken with the same relative detector pose as the original X-ray image and a plurality of non-anatomical markers inserted between the X-ray source and the X-ray detector. In particular, said non-anatomical markers are inserted at predetermined positions. From the locations of the non-anatomical markers in the calibration X-ray image, the relative detector pose can be determined, e.g., by ray-tracing.

Alternatively or additionally, the information relating to the relative detector pose is obtained by performing an analysis of collimation borders of the X-ray beams on the X-ray detector. Given the known collimation applied to the X-ray source, the X-ray beams corresponding to the collimation borders are known. Hence, for example by ray-tracing, the relative detector pose can be obtained by analyzing the collimation borders on the original X-ray image.

Alternatively or additionally, the information relating to the relative detector pose is obtained by comparing the original X-ray image to one or more further X-ray images taken in a longitudinal study. Here, a mapping from the one or more further X-ray images to the original X-ray image can be performed, in particular of recognised structures or sub-structures, yielding one or more known points in the original X-ray image which can then be used to determine the information relating to the relative detector pose.

Alternatively or additionally, the information relating to the relative detector pose is obtained by a direct geometric determination based on at least one image obtained by a stereo camera and/or a depth camera, which provide three-dimensional location information of characteristic points on the X-ray detector and/or the X-ray source. Given the three-dimensional location information of these characteristic points, the relative detector pose can be determined by simple geometric considerations. In particular, the stereo camera and/or the depth camera may be mounted at the X-ray source, such that the three-dimensional location information is directly obtained in a coordinate frame fixed to the X-ray source.

According to an embodiment, the computing unit is configured to interactively obtain the point of interest by user input. In particular, said user input may be performed by a pointing device, pointing to the point of interest, or by touching a touch screen at the location where the point of interest is displayed. Also, the X-ray image processing system may constantly update, in particular in real-time, the transformed X-ray image in dependence on the chosen point of interest.

According to an embodiment, the computing unit is configured to obtain the point of interest by finding a pre-determined or determinable anatomical structure using computer vision. The anatomical structure of interest may be pre-determined by meta-data corresponding to the original X-ray image. Alternatively, a user, in particular a radiologist, may choose the anatomical structure of interest from a list of possible anatomical structures. The computer vision then finds the anatomical structure of interest in the original X-ray image and determines the point of interest. Said computer vision may be implemented, for example, by trained machine learning systems, for example by trained deep learning models. For the training of said models, annotated X-ray images are used, wherein the annotation comprises at least the name and the location of the anatomical structure. Hence, such a trained deep machine learning system can provide the location of the point of interest given an X-ray image and the name of the desired anatomical structure.

According to an embodiment, the perspective transformation comprises a mapping of image points of the original X-ray image to a virtual X-ray detector that is perpendicular to an X-ray beam from the X-ray source to the point of interest. This is a simple projective transformation that can be performed very quickly, even by standard computing systems.

In another aspect of the present invention, a X-ray imaging system is provided. Said X-ray imaging system comprises an X-ray source, an X-ray detector, an X-ray image processing system according to the above description and a display configured to receive and display the transformed X-ray image. In particular, said X-ray imaging system provides transformed X-ray images with perspective distortions minimized at the point of interest and in a region surrounding the point of interest.

According to an embodiment, the X-ray imaging system further comprises a toggle button configured to switch between displaying the original X-ray image and the transformed X-ray image. Using this toggle button, a user, in particular a radiologist, can ensure that he or she does not miss any information by viewing the original X-ray image and can have an optimized view of the point of interest and the region surrounding the point of interest by viewing the transformed X-ray image.

In another aspect of the present invention, a method for processing an X-ray image is provided. According to the method, at least one original X-ray image taken by an X-ray imaging system is obtained. The X-ray imaging system comprises an X-ray source and an X-ray detector. Further, information relating to the relative detector pose of the X-ray detector relative to the X-ray source and a point of interest in the original X-ray image are obtained. Then, a perspective transformation is applied to the original X-ray image to obtain a transformed X-ray image, wherein the transformed X-ray image corresponds to a perspective where the X-ray detector is perpendicular to an X-ray beam from the X-ray source to the point of interest. In particular, the method provides a transformed X-ray image with perspective distortions minimized at the point of interest and in a region surrounding the point of interest. Further advantages are given in the above description.

According to an embodiment, the information relating to the relative detector pose is obtained by one of the following: an external input; an interactive user input; an analysis of a calibration X-ray image taken with the same relative detector pose as the original X-ray image and a plurality of non-anatomical markers inserted between the X-ray source and the X-ray detector; an analysis of collimation borders of the X-ray beams on the X-ray detector; a comparison to one or more further X-ray images taken in a longitudinal study; and a direct geometric determination based on at least one image obtained by a stereo camera and/or a depth camera. Detailed descriptions of said options and further advantages are given in the above description.

According to an embodiment, the point of interest is obtained interactively by user input. A detailed description and advantages of the user input of the point of interest are given in the above description.

According to an embodiment, the point of interest is obtained by finding a pre-determined or determinable anatomical structure using computer vision. A detailed description and advantages of determining the point of interest using computer vision are given in the above description.

According to an embodiment, the perspective transformation comprises a mapping of image points of the original X-ray image to a virtual X-ray detector that is perpendicular to an X-ray beam from the X-ray source to the point of interest. A detailed description and advantages of this method step for applying a perspective transformation are given in the above description.

It shall be understood that a preferred embodiment of the invention can also be any combination of the dependent claims with the respective independent claim.

These and other aspects of the invention will be apparent from and elucidated with reference to the embodiments described hereinafter.

### BRIEF DESCRIPTION OF THE DRAWINGS

In the following, preferred embodiments of the invention will be described, by way of example only, and with reference to the drawings in which:
Fig. 1 shows a schematic view of an embodiment of an X-ray imaging system;
Fig. 2 shows an example of a perspective transformation; and
Fig. 3 shows another example of a perspective transformation.

### DETAILED DESCRIPTION OF EMBODIMENTS

Like numbered elements in these figures are either equivalent elements or perform the same function. Elements which have been discussed previously will not necessarily be discussed in later figures if the function is equivalent.

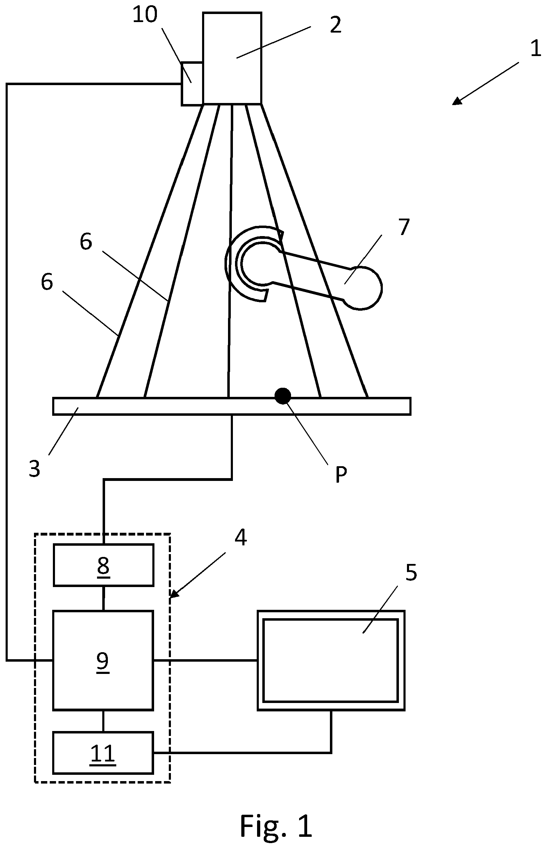

Fig. 1 shows a schematic view of an embodiment of an X-ray imaging system 1. The X-ray imaging system 1 comprises an X-ray source 2, an X-ray detector 3, and X-ray image processing system 4 and a display 5.

The X-ray source 2 is adapted to emit X-ray beams 6 and the X-ray detector 3 is adapted to detect said X-ray beams 6. An anatomical structure 7 between the X-ray source 2 and the X-ray detector 3 will cause attenuation of the X-ray beams 6, which will be detected by the X-ray detector 3.

The X-ray detector 3 is connected to an X-ray image input interface 8 of the X-ray image processing system 4. Said connection may be a wired or a wireless connection. A computing unit 9 of the X-ray image processing system 4 obtains an original X-ray image from the X-ray detector 3 via the X-ray image input interface 8.

The computing unit 9 further obtains information relating to the relative detector pose, i.e., detector position and orientation, of the X-ray detector 3 relative to the X-ray source 2. In the embodiment of Fig. 1, the information relating to the relative detector pose is obtained by a direct geometric determination based on an image taken by a depth camera 10 that is located at the X-ray source 2.

In alternative embodiments, which are not shown here, the information relating to the relative detector pose may be obtained by a direct geometric determination based on an image taken by a stereo camera. Alternatively, the information relating to the relative detector pose can be obtained through an external input, e.g., as information provided by the X-ray imaging system 1 having a fixed X-ray source 2 and X-ray detector 3. Yet alternatively, the information relating to the relative detector pose can be obtained by an interactive user input, where the user is in particular an experienced radiologist who can estimate the geometric distortions based on the X-ray image seen. Yet alternatively, an analysis of a calibration X-ray image taken with the same relative detector pose as the original X-ray image and a plurality of non-anatomical markers inserted instead of the anatomical structure 7 can be used to obtain the relative detector pose. Other alternatives to obtain the relative detector pose are an analysis of collimation borders of the X-ray beams 6 on the X-ray detector 3 and a comparison to one or more further X-ray images taken in a longitudinal study.

The computing unit 9 also obtains a point of interest P in the original X-ray image. In the embodiment of Fig. 1, the point of interest P is provided by a user by identifying the position on the touch display 5.

Alternatively, the user may use a pointing device to identify the point of interest P in the original X-ray image. Yet alternatively, the point of interest P may be obtained by finding a pre-determined or determinable anatomical structure 7 using computer vision. In this alternative, computer vision may be based on a trained machine learning system such as a deep neural network. For training, annotated X-ray images are used, wherein the annotation contains a name of an anatomical structure and the corresponding location of the point of interest in the X-ray image.

The computing unit 9 then applies a perspective transformation to the original X-ray image to obtain a transformed X-ray image, such that the transformed X-ray image corresponds to a perspective where the X-ray detector 3 is perpendicular to an X-ray beam 6 from the X-ray source 2 to the point of interest P. Hence, the perspective distortions at the point of interest P and the region around the point of interest P are minimized in the transformed X-ray image.

The computing unit 9 then transmits the transformed X-ray image to an X-ray image output interface 11 of the X-ray image processing system 4. The X-ray image output interface 11 then transfers the transformed X-ray image to the display 5 where it is displayed.

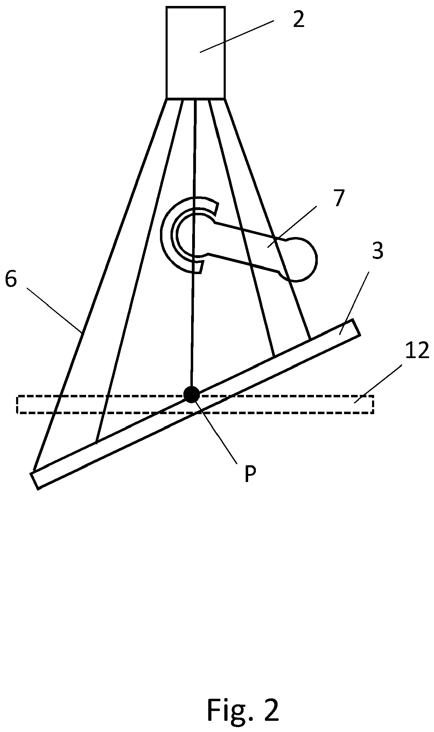

Fig. 2 shows an example of a perspective transformation where the X-ray detector 3 is placed such that the point of interest (corresponding to the center of the joint 7) appears at the center of the detector. However, the detector is tilted with respect to a plane perpendicular to the X-ray beam 6 from the X-ray source 2 to the point of interest P. Hence, the perspective transformation comprises a mapping of image points of the original X-ray image to a virtual X-ray detector 12, wherein the virtual X-ray detector 12 coincides with the X-ray detector 3 at the point of interest P and is arranged such that it is perpendicular to the X-ray beam 6 from the X-ray source 2 to the point of interest P. The resulting image will have minimal perspective distortions at the point of interest P and hence also in the region surrounding the point of interest P.

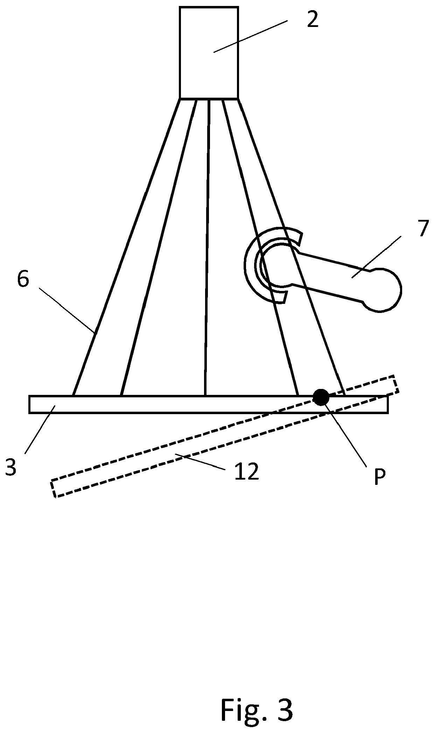

Fig. 3 shows another example of a perspective transformation where the X-ray detector 3 is placed such that it is perpendicular to a central X-ray beam 6, but the point of interest P is located towards the edge of the X-ray detector 3. Again, the perspective transformation comprises a mapping of image points of the original X-ray image to the virtual X-ray detector 12, wherein the virtual X-ray detector 12 coincides with the X-ray detector 3 at the point of interest P and is arranged such that it is perpendicular to the X-ray beam 6 from the X-ray source 2 to the point of interest P. The resulting image will have minimal perspective distortions at the point of interest P and hence also in the region surrounding the point of interest P.

While the invention has been illustrated and described in detail in the drawings and foregoing description, such illustration and description are to be considered illustrative or exemplary and not restrictive; the invention is not limited to the disclosed embodiments.

Other variations to the disclosed embodiments can be understood and effected by those skilled in the art in practicing the claimed invention, from a study of the drawings, the disclosure, and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measured cannot be used to advantage. Any reference signs in the claims should not be construed as limiting the scope.

### LIST OF REFERENCE SIGNS:

- 1: X-ray imaging system
- 2: X-ray source
- 3: X-ray detector
- 4: X-ray image processing system
- 5: Display
- 6: X-ray beam
- 7: Anatomical structure
- 8: X-ray image input interface
- 9: Computing unit
- 10: Depth camera
- 11: X-ray image output interface
- 12: Virtual X-ray detector

- P: Point of interest

## Claims

1. X-ray image processing system comprising:
an X-ray image input interface (8) configured to receive original X-ray images taken by an X-ray imaging system (1) comprising an X-ray source (2) and an X-ray detector (3);
a computing unit (9) connected to the X-ray image input interface (8) and configured to obtain at least one original X-ray image from the X-ray image input interface (8);
obtain information relating to the relative detector pose of the X-ray detector (3) relative to the X-ray source (2);
obtain a point of interest (P) in the original X-ray image; and
apply a perspective transformation to the original X-ray image to obtain a transformed X-ray image wherein the transformed X-ray image corresponds to a perspective where the X-ray detector (3) is perpendicular to an X-ray beam (6) from the X-ray source (2) to the point of interest (P); and
an X-ray image output interface (11) configured to transmit the transformed X-ray image.

2. X-ray image processing system according to claim 1, wherein the information relating to the relative detector pose comprises a relative position and a relative orientation of the X-ray detector (3) relative to the X-ray source (2).

3. X-ray image processing system according to claim 1 or 2, wherein the information relating to the relative detector pose has been provided by external input.

4. X-ray image processing system according to any of claims 1 to 3, wherein the computing unit (9) is configured to interactively obtain the information relating to the relative detector pose, in particular a direction and an amount of an X-ray detector (3) tilt, by user input.

5. X-ray image processing system according to any of claims 1 to 4, wherein the computing unit (9) is configured to obtain the information relating to the relative detector pose by at least one of the following:
an analysis of a calibration X-ray image taken with the same relative detector pose as the original X-ray image and a plurality of non-anatomical markers inserted between the X-ray source (2) and the X-ray detector (3);
an analysis of collimation borders of the X-ray beams (6) on the X-ray detector (3);
a comparison to one or more further X-ray images taken in a longitudinal study; and
a direct geometric determination based on at least one image obtained by a stereo camera and/or a depth camera (10).

6. X-ray image processing system according to any of claims 1 to 5, wherein the computing unit (9) is configured to interactively obtain the point of interest (P) by user input.

7. X-ray image processing system according to any of claims 1 to 6, wherein the computing unit (9) is configured to obtain the point of interest (P) by finding a pre-determined or determinable anatomical structure (7) using computer vision.

8. X-ray image processing system according to any of claims 1 to 7, wherein the perspective transformation comprises a mapping of image points of the original X-ray image to a virtual X-ray detector (12) that is perpendicular to an X-ray beam (6) from the X-ray source (2) to the point of interest (P).

9. X-ray imaging system comprising an X-ray source (2), an X-ray detector (3), an X-ray image processing system (4) according to any one of claims 1 to 8, and a display (5) configured to receive and display the transformed X-ray image.

10. X-ray imaging system according to claim 9, further comprising a toggle button configured to switch between displaying the original X-ray image and the transformed X-ray image.

11. Method for processing an X-ray image, comprising:
obtaining at least one original X-ray image taken by an X-ray imaging system (1) comprising an X-ray source (2) and an X-ray detector (3);
obtaining information relating to the relative detector pose of the X-ray detector (3) relative to the X-ray source (2);
obtaining a point of interest (P) in the original X-ray image; and
applying a perspective transformation to the original X-ray image to obtain a transformed X-ray image wherein the transformed X-ray image corresponds to a perspective where the X-ray detector (3) is perpendicular to an X-ray beam (6) from the X-ray source (2) to the point of interest (P).

12. Method according to claim 11, wherein the information relating to the relative detector pose is obtained by at least one of the following:
an external input;
an interactive user input;
an analysis of a calibration X-ray image taken with the same relative detector pose as the original X-ray image and a plurality of non-anatomical markers inserted between the X-ray source (2) and the X-ray detector (3);
an analysis of collimation borders of the X-ray beams (6) on the X-ray detector (3);
a comparison to one or more further X-ray images taken in a longitudinal study; and
a direct geometric determination based on at least one image obtained by a stereo camera and/or a depth camera (10).

13. Method according to claim 11 or 12, wherein the point of interest (P) is obtained interactively by user input.

14. Method according to claim 11 or 12, wherein the point of interest (P) is obtained by finding a pre-determined or determinable anatomical structure (7) using computer vision.

15. Method according to any of claims 11 to 14, wherein the perspective transformation comprises a mapping of image points of the original X-ray image to a virtual X-ray detector (12) that is perpendicular to an X-ray beam (6) from the X-ray source (2) to the point of interest (P).
